# EUROPEAN PATENT SPECIFICATION

(11) **EP 0 976 701 B1**
(45) Date of publication and mention of the grant of the patent: **13.01.2010**
(21) Application number: 96938454.4
(22) Date of filing: 13.11.1996
(51) Int. Cl.: C07B 61/00, G06F 17/50, C07K 1/00

(54) **DESIGN METHOD OF PHYSIOLOGICALLY ACTIVE COMPOUND**
VERFAHREN ZUM ENTWURF EINER PHYSIOLOGISCH AKTIVEN VERBINDUNG
PROCEDE DE MISE AU POINT DE COMPOSE PHYSIOLOGIQUEMENT ACTIF

(30) Priority: 13.11.1995 JP 29418995
(43) Date of publication of application: 02.02.2000
(73) Proprietor: Itai, Akiko, Bunkyo-ku, Tokyo 113 (JP); Institute of Medicinal Molecular Design, Inc., Tokyo 113-0033 (JP)
(72) Inventor: ITAI, Akiko, Tokyo 113 (JP); TOMIOKA, Nobuo, Tokyo 113 (JP)
(74) Representative: Price, Nigel John King
(86) International application number: PCT/JP1996/003325
(87) International publication number: WO 1997/018180

(56) References cited:
- WANG S, ZAHAREVITZ DW, SHARMA R, ET AL: "The discovery of novel, structurally diverse protein kinase C agonists through computer 3D-database pharmacophore search. Molecular modeling studies" JOURNAL OF MEDICINAL CHEMISTRY, vol. 37, 29 April 1994 (1994-04-29), pages 4479-4489, XP002366753
- HO C M W ET AL: "FOUNDATION: A PROGRAM TO RETRIEVE ALL POSSIBLE STRUCTURES CONTAINING A USER-DEFINED MINIMUM NUMBER OF MATCHING QUERY ELEMENTS FOR THREE-DIMENSIONAL DATABASES" JOURNAL OF COMPUTER-AIDED MOLECULAR DESIGN, ESCOM SCIENCE PUBLISHERS BV, XX, vol. 7, no. 1, February 1993 (1993-02), pages 3-22, XP008059321 ISSN: 0920-654X
- MARTIN Y C: "3D DATABASE SEARCHING IN DRUG DESIGN" JOURNAL OF MEDICINAL CHEMISTRY, AMERICAN CHEMICAL SOCIETY. WASHINGTON, US, vol. 35, no. 12, 12 June 1992 (1992-06-12), pages 2145-2154, XP000676670 ISSN: 0022-2623
- DRIE VAN J H ET AL: "ALADDIN: AN INTEGRATED TOOL FOR COMPUTER-ASSISTED MOLECULAR DESIGN AND PHARMACOPHORE REGOGNITION FROM GEOMETRIC, STERIC, AND SUBSTRUCTURE SEARCHING OF THREE-DIMENSIONAL MOLECULAR STRUCTURES" JOURNAL OF COMPUTER-AIDED MOLECULAR DESIGN, ESCOM SCIENCE PUBLISHERS BV, vol. 3, no. 3, 1989, pages 225-251, XP002947281 ISSN: 0920-654X
- SHERIDAN R P; RUSINKO A 3RD; NILAKANTAN R R P; RUSINKO A 3RD; NILAKANTAN R; VENKATARAGHAVAN R: "Searching for pharmacophores in large coordinate data bases and its use in drug design." PROC NATL ACAD SCI USA, vol. 86, no. 20, October 1989 (1989-10), pages 8165-8169, XP002366754
- PROC. NATL. ACAD. SCI. USA, Vol. 87, 1990, R.L. DESJARLAIS et al., "Structure-based Design of Nonpeptide Inhibitors Specific for the Human Immunodeficiency Virus 1 Protease", pages 6644-6648. XP000605236
- TETRAHEDRON COMPUTER METHODOLOGY, Vol. 3, No. 6C, 1990, JOSEPH B. MOON et al., "3D Database Searching and de Novo Construction Methods in Molecular Design", pages 697-711. XP000618577
- SHOICHI SAITO, AKIKO ITAI: 'RATIONAL DRUG DESIGN BASED ON THE 3D STRUCTURE OF INFLUENZA SIALIDASE' EXPERIMENTAL MEDICINE vol. 12, no. 4, March 1994, pages 68 (444) - 70 (446), XP002999231
- AKIKO ITAI: 'Structural Analysis of Malarial DHFR and Inhibitor Design, Chemistry of Natural Super Molecules' REPORT IN FISCAL YEAR HEISEI 6 June 1995, THE INTENSIVE FIELD RESEARCH MADE BY A SUBSIDY OF THE MINISTRY OF EDUCATION, pages 212 - 215
- NOBUO TOMIOKA, AKIKO ITAI: 'Current Status and Prospects for a Drug Design Method Aided by Means of a Computer' INFECTION, INFLAMMATION AND TISSUE IMMUNITY vol. 25, no. 2, June 1995, pages 56 (128) - 59 (131), XP002999232
- WILLETT P.: 'SEARCHING FOR PHARMACOPHORIC PATTERNS IN DATABASES OF THREE-DIMENSIONAL CHEMICAL STRUCTURES' JOURNAL OF MOLECULAR RECOGNITION vol. 8, 01 January 1995, HEYDEN & SON LTD., LONDON, GB, pages 290 - 303, XP000885656
- BARNARD J.M.: 'SUBSTRUCTURE SEARCHING METHODS: OLD AND NEW' JOURNAL OF CHEMICAL INFORMATION AND COMPUTER SCIENCES vol. 33, 01 July 1993, pages 532 - 538, XP000611888
- BARNARD J.M.: 'CLUSTERING OF CHEMICAL STRUCTURES ON THE BASIS OF TWO-DIMENSIONAL SIMILARITY MEASURES' JOURNAL OF CHEMICAL INFORMATION AND COMPUTER SCIENCES vol. 32, 01 November 1992, pages 644 - 649, XP002910453
- PEPPERRELL C.A.; WILLETT P.; TAYLOR R.: 'IMPLEMENTATION AND USE OF AN ATOM-MAPPING PROCEDURE FOR SIMILARI TY SEARCHING IN DATABASES OF 3-D CHEMICAL STRUCTURES' TETRAHEDRON COMPUTER METHODOLOGY vol. 3, 1990, pages 575 - 593, XP008078645
- HAGADONE T.R.: 'MOLECULAR SUBSIMILARITY SEARCHING: EFFICIENT RETRIEVAL IN TWO-DIMENSIONAL STRUCTURE DATABASES' JOURNAL OF CHEMICAL INFORMATION AND COMPUTER SCIENCES vol. 32, 01 January 1992, pages 515 - 521, XP008078626
- BRINT A.T.; WILLETT P.: 'PHARMACOPHORIC PATTERN MATCHING IN FILES OF 3-D CHEMICAL STRUCTURES: COMPARISON OF GEOMETRIC SEARCHING ALGORITHMS' JOURNAL OF MOLECULAR GRAPHICS vol. 5, no. 1, 01 January 1987, pages 49 - 56, XP008078630
- BEMIS G.W.; KUNTZ I.D.: 'A FAST AND EFFICIENT METHOD FOR 2D AND 3D MOLECULAR SHAPE DESCRIPTION' JOURNAL OF COMPUTER-AIDED MOLECULAR DESIGN vol. 6, 01 January 1992, pages 607 - 628, XP008078649

## Description

### Technical Field

The present invention relates to a method for selecting lead compounds useful for molecular design of physiologically active compounds such as drugs and agricultural chemicals from a database which contains information of compounds by using a computer.

### Prior Art

In order to create useful drugs, agricultural chemicals and the like, it is essential to use a lead compound that has been already confirmed to have a desired physiological activity and which should be a starting point of various chemical modifications. On the other hand, it has been known that a physiologically active compound interacts specifically with a certain polymer in the living body (it is herein referred to as a "biopolymer" or "receptor" as the case may be). However, any logical method for creating a lead compound has not yet been known. Therefore, in general, lead compounds are taken from known biological substances acting in the living body, from compounds for which desired physiological activity has been discovered by chance or by random screening, or from compounds whose chemical structures have been somewhat modified from those described above. However, various computerized methods for creating lead compounds have been developed in recent years, and thus it has been becoming possible to logically create lead compounds by computerized design of a structure which satisfies requirements including structural factors and interaction scheme such as hydrogen bonds necessary for the expression of the intended physiological activity, when such requirements can be estimated in advance.

Nowadays, three-dimensional structures of many biopolymers have been already elucidated, and many three-dimensional structures of complexes of a low molecular weight compound such as an enzyme inhibitor (as used herein, "ligand" means a low molecular weight compound generally having a molecular weight of 1,000 or less capable of binding to a biopolymer) and a biopolymer have also been reported. Based on these studies, it has been revealed that, in order to be a ligand, a candidate compound must have its molecular shape and local physicochemical properties complementary with those of the drug binding site, while it needs not to resemble a intrinsic ligand or a known ligand whose activity have been found by chance in its skeletal structure and its arrangement of substituent groups. Many chemical structures that can become a ligand of a specific biopolymer are considered to exist, and by designing or searching for such structures by a computer based on the information of biopolymers and known ligands, it has become possible to create novel lead compounds efficiently. In general, for predicting whether a compound has a desired physiological activity, one can use criteria whether the compound can bind stably to the binding site of the biopolymer with good fitness. When information about three-dimensional structure of the biopolymer is not available, one can use structural information of drug molecules known to be capable of binding to the biopolymer and can use criteria whether kinds and relative three-dimensional positions of functional groups correspond well between the compound and the drug molecules.

As a computerized method for finding compounds meeting such requirements as mentioned above, one can consider a method of automatically designing ligand compounds computationally (automatic structure construction method) and a method of searching for desired compounds from a database of three-dimensional structures. In the automatic structure construction method, the algorithm to be used may be different depending on what kind of information can be utilized. For the case where three-dimensional structure of the target biopolymer is available, the present inventors have successfully developed a method for building ligand structures by generating atoms one by one using random numbers and force fields while enabling stable binding to the specified ligand binding site and forming many hydrogen bonds and the like (program LEGEND, Nishibata, Y. and Itai, A., Tetrahedron, 47, pp.8985-8990, 1991; Nishibata, Y. and Itai, A., J. Med. Chem., 36, pp.2921-2928, 1993).

There has also been known a method for suggesting possible ligand structures which stores partial structures frequently found in drug compounds in a program as fragment structures, sequentially fits those structures to a ligand binding site divided into several parts, and finally connects fragments that can fit each part of the site with acceptable linking atomic groups (Boehm, J.H. et al., J. Comput. Aided Molecular Design, 6, pp.593-606, 1992). The advantage of these automatic structure construction methods is that they can broadly suggest various desirable structures that meet the requirements for having a physiological activity regardless of whether compounds having such structures are known or unknown. However, there are problems that possibility of obtaining a chemical substance having the same structure as output from a computer is quite low, and that the compound needs to be newly synthesized in most cases. Moreover, the presented structure of the compound may not be preferable at all from a standpoint of synthesis, although it may be excellent from a standpoint of fitness to the drug binding site of the receptor (biopolymer).

On the other hand, advantage of the database method is that one can obtain the desired compound immediately and can evaluate its biological activity without an effort of synthesis if a compound satisfying the requirement is retrieved by searching an in-house or commercial database of available compounds. Accordingly, the database method has advantages of saving labor and time required for synthesis, and of enabling assay of a large number of compounds at a time. After selection of compounds that exhibit strong activity to some extent and that are easy to be synthesized, and after modification of the structures for improving their activity and/or physical properties, one can intend to an extensive synthetic study.

Most of the compound databases that are generally available store atomic types and atomic coordinate of each atom and mode of covalent bonds (covalently bonded atom pairs and bond types) as information about each compound. Based on this information, the database is utilized for retrieving compounds having a specific molecular skeleton, partial structure, or atom-connection pattern. However, in order to find a novel lead compound that can be a ligand of a certain biopolymer, it is necessary to search three-dimensional structure database based on a three-dimensional structure of the biopolymer or based on three-dimensional structures of known ligands. In the three-dimensional structure search, handling of conformational freedom of compounds, in particular, conformational freedom of ring structures, is an extremely difficult problem, and enormous computation time is required for testing requirements for the activity while considering all possible conformations of each compound. Moreover, still longer computation time is required if one needs to consider problems of absolute configurations and relative configurations of compounds, and therefore it is not a practical method for searching a database containing several tens of thousands to several millions of compounds.

Accordingly, the object of the present invention is to provide a method for searching for lead compounds which solves the problems of the prior art mentioned above. WANG S, ZAHAREVITZ DW, SHARMA R, ET AL: "The discovery of novel, structurally diverse protein kinase C agonists through computer 3D-database pharmacophore search. Molecular modeling studies" JOURNAL OF MEDICINAL CHEMISTRY, vol. 37, 29 April 1994 (1994-04-29), pages 4479-4489, XP002366753, discloses a program for performing a pharmacophore-based search of a 3D-database for potential lead compounds, the search being based on the distances between atoms in the pharmacophore, together with sub-structural requirements such as the presence of certain groups or atoms at certain positions.

HO C M W ET AL.: "FOUNDATION: A PROGRAM TO RETRIEVE ALL POSSIBLE STRUCTURES CONTAINING A USER-DEFINED MINIMUM NUMBER OF MATCHING QUERY ELEMENTS FOR THREE-DIMENSIONAL DATABASES" JOURNAL OF COMPUTER-AIDED MOLECULAR DESIGN, ESCOM SCIENCE PUBLISHERS BV, XX, vol. 7, no. 1, February 1993 (1993-02) pages 3-22, XP008059321 ISSN: 0920-654X, discloses a program for searching a 3D-database using a user-defined query containing a combination of a user-specified minimum number of matching elements. The queries consist of three-dimensional co-ordinance of atoms and/or bonds.

MARTIN Y C: "3D DATABASE SEARCHING IN DRUG DESIGN" JOURNAL OF MEDICINAL CHEMISTRY, AMERICAN CHEMICAL SOCIETY. WASHINGTON, US, vol. 35, no. 12, 12 June 1992 (1992-06-12), pages 2145-2154, XP000676670 ISSN: 0022-2623, is a review of various 3D database searching techniques in medicinal chemistry.

Further methods of searching compound and structure databases are disclosed in:
WILLETT P: "SEARCHING FOR PHARMACOPHORIC PATTERNS IN DATABASES OF THREE-DIMENSIONAL CHEMICAL STRUCTURES" JOURNAL OF MOLECULAR RECOGNITION, vol. 8, pages 290-303, XP000885656
BARNARD J M: "SUBSTRUCTURE SEARCHING METHODS: OLD AND NEW" JOURNAL OF CHEMICAL INFORMATION AND COMPUTER SCIENCES, vol. 33, 1 July 1993 (1993-07-01), pages 532-538, XP000611888
BARNARD J M: "CLUSTERING OF CHEMICAL STRUCTURES ON THE BASIS OF TWO-DIMENSIONAL SIMILARITY MEASURES" JOURNAL OF CHEMICAL INFORMATION AND COMPUTER SCIENCES, vol. 32, 1 November 1992 (1992-11-01), pages 644-649, XP002910453
PEPPERRELL C A, WILLETT P; TAYLOR R: "IMPLEMENTATION AND USE OF AN ATOM-MAPPING PROCEDURE FOR SIMILARITY SEARCHING IN DATABASES OF 3-D CHEMICAL STRUCTURES" TETRAHEDRON COMPUTER METHODOLOGY, vol. 3, 1990, pages 575-593, XP008078645
HAGADONE T R: "MOLECULAR SUBSIMILARITY SEARCHING: EFFICIENT RETRIEVAL IN TWO-DIMENSIONAL STRUCTURE DATABASES" JOURNAL OF CHEMICAL INFORMATION AND COMPUTER SCIENCES, vol. 32, pages 515-521, XP008078626
BRINT A T; WILLETT P: "PHARMACOPHORIC PATTERN MATCHING IN FILES OF 3-D CHEMICAL STRUCTURES: COMPARISON OF GEOMETRIC SEARCHING ALGORITHMS" JOURNAL OF MOLECULAR GRAPHICS, vol. 5, no. 1, pages 49-56, XP008078630
BEMIS G W; KUNTZ I D: "A FAST AND EFFICIENT METHOD FOR 2D AND 3DMOLECULAR SHAPE DESCRIPTION" JOURNAL OF COMPUTER-AIDED MOLECULAR DESIGN, vol. 6, pages 607-628, XP008078649

A novel method has been developed for creating lead compounds which takes the advantages of both the automatic structure construction method and the database method, and successfully developed a method for efficiently selecting lead compounds from a database which solves the problems of both the methods.

The present invention provides a computer-based method for selecting lead-candidate compounds capable of binding to a receptor bipolymer as defmed in claim 1.

The invention will be further described by way of example with reference to the accompanying drawings, in which:-
Figure 1 represents an algorithm for a preferred embodiment of the method of the present invention comprising the steps of (a) to (f).
Figure 2 represents a detailed algorithm of a preferred embodiment of the method of the present invention. In this figure, S represents a step.
Figure 3 represents chemical structures of a part of the compounds selected by the method of the present invention from a compound database, Available Chemical Directory, as lead-candidate compounds capable of binding to a biopolymer, dihydrofolate reductase, along with their relation to the query molecules.
Figure 4 represents comparison of binding schemes to a ligand binding site (cavity) of the biopolymer with respect to the preferred lead-candidate compounds selected in the third screening and the query molecules. In this figure, cage-like indications represent regions into which atoms can enter, and molecular structures of the biopolymer are indicated with normal lines, and the structures of query molecules (left) and preferred lead-candidate compounds (right) are indicated with bold lines. Hydrogen bonds between the ligands and the biopolymer are indicated with dotted lines.

The database which can be used for the method of the present invention stores chemical structures of two or more, preferably numerous, compounds in a computer-readable format, and preferably contains information about atomic types and covalent bond mode of the stored compounds. The term "atomic type" is used herein for including any methods for classifying atoms such as a classification method fractionalized by hybridization status in view of a type of an element. The term "covalent bond mode (mode of covalent bond)" used herein includes information of counterpart atom covalently bonded to a certain atom indicated by input order numbers of the atoms and kind of the chemical bond such as a single bond or a double bond.

In general, a database in a format containing two-dimensional coordinate information for visualizing compounds on a display in addition to the above-mentioned information (a database in such a format is proposed by MDL Information Systems, Inc. as "Molfile" format) can be utilized. For example, as a database storing commercially available compounds, Available Chemicals Directory (MDL Information Systems, Inc.) can be utilized. Further, databases offered by reagent-selling companies (such as those offered by companies including Maybridge, SPECS, Peakdale, Labotest, and Bionet), a database storing chemical structures and literature information described in Chemical Abstracts (Chemical Abstracts File), databases storing virtual compound structures and the like can be utilized. A method utilizing a database from which three-dimensional coordinate information of compounds is available (Cambridge Structural Database etc.) is a preferred embodiment of the present invention.

In order to select lead-candidate compounds capable of binding to a receptor biopolymer from such a database as mentioned above, the following steps may be performed (a) a step of selecting lead-candidate compounds by matching one or more query molecules capable of binding to the biopolymer with compounds stored in a database based on information about atomic types and covalent bond mode of the query molecules.

As the query molecules for screening the database, one or more kinds of known ligands known to be capable of binding to the biopolymer can be used. Alternatively, structures of one or more query compounds capable of binding to the biopolymer may be constructed by an automatic structure construction method (step (b)). When it is difficult to utilize information of known ligands as the query molecules, it is generally preferred to perform a method comprising the step (b).

The above step (b) is generally performed by constructing novel ligand structures capable of binding to a specific biopolymer based on available information about three-dimensional structure for the biopolymer and/or known ligands capable of binding to the biopolymer. As the automatic structure construction method used in the step (b), any method can be used so long as it can afford construction of ligands capable of binding to the biopolymer by calculation based on the information about three-dimensional structure for the biopolymer and/or known ligands capable of binding to the biopolymer. As examples of such automatic structure construction methods, methods and the like which involve locating atoms one by one can be mentioned as follows; LEGEND (Nishibata, Y. and Itai, A., Tetrahedron, 47, pp.8985-8990, 1991; Nishibata, Y. and Itai, A., J. Med. Chem., 36, pp.2921-2928, 1993), CONCEPTS (Pearlman, D.A. and Murcko, M.A., J. Comp. Chem., 14, pp.1184-1193, 1993), MCDNLG (Gehlhaar, D.K. et al., J. Med. Chem., 38, pp.466-472, 1995). Alternatively, methods which involve linking fragments such as LUDI (Boehm, H.-J., J. Comput.-Aided Mol. Design, 6, pp.61-78, 1992), GroupBuild (Rotstein, S.H. and Murcko, M.A., J. Med. Chem., 36, pp.1700-1710, 1993), SPROUT (Gillet, V. et al., J. Comput.-Aided Mol. Design, 7, pp.127-153, 1993), HOOK (Eisen, M.B. et al., PROTEINS: Struct. Func. Genet., 19, pp.199-221, 1994) and the like can also be utilized.

It is also possible to construct ligand structures by extracting functional groups and their arrangement essential for binding to a biopolymer based on three-dimensional structures of one or more known ligands capable of binding to the biopolymer, and generating stable skeletal structures that links those functional groups. An example of such a method is known as LINKOR (Inoue, A. et al., The 19th Symposium for Structure-Activity Relationship, subject number 29S23, 1991; Kanazawa, T. et al., 20th Symposium for Structure-Activity Relationship, subject number 27S22, 1992; Takeda, M. et al., 21st Symposium for Structure-Activity Relationship, subject number 26S25, 1993; Japanese Patent Unexamined Publication No. Hei 6-309385/1994; and Japanese Patent Unexamined Publication No. Hei 7-133233/1995), and it can be utilized by those skilled in the art.

As a preferred example of the automatic structure construction method, the algorithm of LEGEND is shown below. LEGEND is a method for constructing ligand structures by generating atoms one by one based on random numbers and molecular force fields while satisfying stableness of the ligand structure both for its intramolecular energy and for its intermolecular energy. For initiating structure construction according to this algorithm, the first atom can be automatically generated at a position where a hydrogen bond can be formed to a hydrogen-bonding atom (anchor atom) in the biopolymer, or alternatively, a partial structure comprising several atoms (seed) which is placed in the binding site of the biopolymer can be used as a starting structure. By using a partial structure important for specific binding to the biopolymer such as those commonly existing in known ligands or a molecular structure predicted to bind specifically to the biopolymer according to docking study, as a starting structure (seed) for the automatic structure construction, structures of other parts can be constructed efficiently.

Ligand-Binding Site in Receptor
Calculate Grid-Point Data
Generate First Atom against
Receptor Anchor Atom
Input Starting Structure (Seed)
Select a Point for Next Atom Addition (Root Atom)
Backtrack
Select Bond Type and Atom Type
Calculate Atom Position
Conversion to Heteroatom (based on
Hydrogen-Bond Flag or Electrostatic-Potential)
Energy Evaluation (Intramolecular + Intermolecular)
One Atom Added
Specified Number of Atoms Generated
Completion of Structure
Supplement Hydrogen and
Aromatic-Ring Atoms
Optimize Structure
Selection of Structure
Number of Hydrogen Bonds
Number of Rings
Intra- and Intermolecular Energies
etc.
Output Structure

After preparing one or more query molecules capable of binding to a biopolymer, structural information of each query molecule is utilized for the subsequent screening. As information of the query molecules, information about atomic types and mode of covalent bonds as well as information about atomic coordinates (information including values of X, Y and Z of a three-dimensional coordinate represented by orthogonal coordinate system) and the like can be utilized. While the number of the query molecules is not limited, it may be desirable that the number of query molecules should be reduced, for example, to around 1-100. As criteria for such reduction, certain numerical criteria as well as other abstract or subjective criteria such as molecular skeletons, flexibility of molecules, and binding schemes to ligand binding sites can be used. For example, when molecular structures output from the program LEGEND are used as query molecules, criteria including intramolecular and intermolecular energy, energy of the whole system, number of hydrogen bonds, hydrogen bonds to specified locations, formation of ionic bonds, number of rings and the like can be employed. The information of the query molecules may be stored in a structure file if necessary.

Then, selection of lead-candidate compounds capable of binding to a biopolymer may be performed by matching of the query molecules with compounds stored in the database (trial compounds) based on the information about atomic types and mode of covalent bonds. The above step (a) may comprise the following steps: (c) a step of first screening by selecting trial compounds based on one or more parameters selected from a group of parameters consisting at least of number of atoms, number of bonds, number of ring structures, number of atoms for each atomic type and molecular weight; and/or (d) a step of second screening by matching of the candidate compounds selected in the first screening step for mode of covalent bond. While a method comprising the steps (c) and (d) will be specifically explained below as a preferred embodiment of the method of the present invention, the method of the present invention is not limited to this method.

First, structure information about every query molecule is read from structure files, and parameters that are used as criteria in the first screening of the step (c) are calculated. As the parameters, one or more of total number of atoms, total number of bonds, number of ring structures, number of atoms for each atomic type, molecular weight and the like can be used, for example. Preferably, two or more kinds of the parameters selected therefrom are appropriately used in combination. Then, data for a compound are read from the database one after another, and for that compound (trial compound), parameters that are computable, preferably all, among those assigned for the query molecules are calculated.

Subsequently, selection of the trial compound is performed by comparing each parameter between each of the query molecules and the trial compound. A trial molecule for which any one of the parameters is too much different from that of the query molecule beyond acceptable criteria is rejected as a candidate for the second screening. For this purpose, it is generally necessary to specify an upper limit and/or a lower limit for each parameter. For example, if the difference of the parameter of total number of atoms is represented as [number of atoms in query molecule] - [number of atoms in trial molecule], and the lower limit of the difference of the parameter is defined as -3 and the upper limit as +2, molecules having number of atoms lower by 3 to higher by 2 compared with the query molecules will be selected. However, there may be parameters which do not require such limits, and such parameters are optionally excluded from selection criteria. As for certain parameters such as number of atoms for each chemical element, selection can be performed by using a secondary parameter such as that derived by adding the number of nitrogen atoms and the number of oxygen atoms.

Then, the second screening by matching of the trial compounds selected in the first screening with the query molecules for the mode of covalent bond can be performed (step (d)). The matching for the mode of covalent bond is an operation wherein, for example, the trial compounds are evaluated by judging which atoms are bonded to which atoms within the molecules, what kind those bonds are (kinds of bond such as single bond, double bond, triple bond and aromatic bond) and the like, and similarity of chemical structure (chemical formula) between trial compound and query molecule is determined by superposing the evaluation results and structural information of the query molecules. For example, this operation is preferably performed by judging similarity of partial structures based on two-dimensional graphs where each atom is represented as a node and each covalent bond is represented as an arc.

That is, if a graph of a trial compound from which one or more nodes and arcs are removed (partial graph) corresponds to a two-dimensional graph of a query molecule, it can be judged that the query molecule is a partial structure of the trial compound. On the other hand, if a partial graph of a query molecule from which one or more nodes and arcs are removed corresponds to a two-dimensional graph of a trial compound, it is judged that the trial compound is a partial structure of the query molecule. For the determination of correspondence of two-dimensional graphs, the algorithm of Ullman (Ullman, J., Assoc. Comput. Mach., 23, p.31, 1976) is preferably used, for example.

In the above-mentioned judgement of correspondence of two-dimensional graphs, correspondence of nodes (kind of atom and/or properties) and/or correspondence of arcs (kind of bond such as single bond, double bond, triple bond, and aromatic bond) can be considered, or alternatively, can be ignored. When such correspondences of kinds and/or properties are considered, the requirements for the correspondences may be loosened optionally as required. For example, several kinds of atoms specified in advance can be regarded to correspond to each other, or a double bond and an aromatic bond can be regarded to correspond to each other.

When the above-mentioned method is used for the second screening, query molecules for which any of the judgements described below have turned out true are selected as the result of the second screening. That is, if the number of atoms in a query molecule is smaller than that of a trial compound, a judgement may be done whether the chemical structure of the query molecule is contained in the trial compound as a partial structure. On the other hand, when the number of atoms in a query molecule is larger than that of a trial compound, a judgement may be done whether the chemical structure of the trial compound is contained in the query molecule as a partial structure.

The query molecules used for each of the above steps contain information about location and/or property of atoms or atomic groups (marker site) that are considered to be essential for effective interaction with the ligand binding site of the biopolymer. For example, when the query molecules have been automatically constructed by using the program LEGEND in the above step (b), partial structures such as functional groups necessary for effective interaction with the ligand binding site of biopolymer are introduced into the query molecules, which are ligands. Such partial structures are precisely selected so that the query molecules can form hydrogen bonds, ionic bonds and the like efficiently and three-dimensionally with the atomic groups present in the ligand binding site of the biopolymer, and that the query molecules can bind strongly to the ligand binding site. Accordingly, by using information about the marker site of the query molecules as a term for the evaluation, the second screening can be performed more efficiently.

As information of such a marker site, relative position of two or more atoms in the query molecules, presence or absence of a specific functional group, hydrogen-bond property (such as hydrogen donor or hydrogen acceptor) of functional groups, property of ionic bond and/or hydrophobic or hydrophilic property of functional group can be utilized as well as a specific partial structure of the query molecules.

By the above-mentioned steps, lead-candidate compounds capable of binding to a receptor biopolymer can be selected from a database containing atomic types and covalent bond modes of compounds as information. For the lead-candidate compounds selected by the above-mentioned steps, it is further possible to select one or more preferred lead-candidate compounds with higher possibility for having a physiological activity by estimating binding schemes of the lead-candidate compounds to the biopolymer based on three-dimensional information of the query molecules and their binding schemes to the biopolymer, and then calculating one or more parameters (for example, interaction energy or number of hydrogen bonds) relating to interaction between the lead-candidate compounds and the biopolymer (third screening step: step (f)). Alternatively, one or more preferred lead-candidate compounds may be selected by estimating a virtual receptor model which represents physicochemical environment of the ligand binding site of the biopolymer based on information about three-dimensional structure of one or more known ligands capable of binding to the biopolymer, and then judging goodness of fit of the lead-candidate compounds selected in the step (a) to the virtual receptor model (third screening step: step (g)).

Because the third screening step requires three-dimensional structure information of the lead-candidate compounds, this step is particularly suitable when the method of the present invention is carried out by using a database from which information of three-dimensional coordinate and the like are available. When information of three-dimensional coordinate for the lead-candidate compounds selected in the second screening is not contained in the database, three-dimensional coordinate are preferably calculated by, for example, methods of CONCORD (TRIPOS Associates Inc.); CONVERTER (BIOSYM/MSI Inc.); and CORINA (Sadowski, J. and Gasteiger, J., Chem. Rev., 93, pp.2567-2581, 1993). For example, when the program LEGEND has been used as the automatic structure construction method, three-dimensional data about the biopolymer, for example, atomic coordinates of the biopolymer and grid-point data representing physicochemical properties of the binding site of the biopolymer and the like can be read for the purpose of the third screening. As the grid-point data, data calculated according to the method of Tomioka et al. can be used (Tomioka, N, and Itai, A., J. Comput. Aided Mol. Design, 8, p.347, 1994).

In order to estimate binding schemes to the biopolymer of the lead-candidate compounds selected in the second screening according to the step (f), any method available for those skilled in the art can optionally be utilized. Preferred method is, for example, a least-squares calculation of interatomic distances of corresponding atoms based on the correspondence of two-dimensional graphs containing information about atoms and covalent bonds, which is used for the second screening. Then, for each atom of the lead-candidate compound superposed onto a query molecule, interaction energy with the biopolymer is determined by referring to neighboring grid-point data, and one or more compounds having interaction energy lower than a specified threshold value can be selected as preferred lead-candidate compounds. For the calculation of the interaction energy, the method of Tomioka et al. (Tomioka, N, and Itai, A., J. Comput. Aided Mol. Design, 8, p.347, 1994) can be employed.

In order to estimate a virtual receptor model according to the step (g), for example, shape and properties of a ligand binding site of the biopolymer may be estimated based on the information of a specific known ligand known to be capable of binding to the biopolymer, or based on the result of superposition of two or more known ligands known to be capable of binding to the biopolymer so that their properties such as shape, hydrogen bonding, electrostatic potential and the like correspond well in the three-dimensional space. As the method for estimating the virtual receptor model, RECEPS (Kato, Y. et al., Tetrahedron Lett., 43, pp.5229-5236, 1987; and Itai, A. et al., "Molecular Superposition for Rational Drug Design" in 3D-QSAR in Drug Design Theory, Methods and Applications," Ed. Kubinyi, H., ESCOM, Netherland, pp.200-225, 1993) can be utilized. This method has an advantage that it can estimate which functional groups in a ligand molecule are essential for binding, in addition to the estimation of virtual receptor model. The lead-candidate compounds selected in the second screening can be fitted to the virtual receptor model estimated by this step, and one or more preferred lead-candidate compounds can be selected by judging goodness of the fitting.

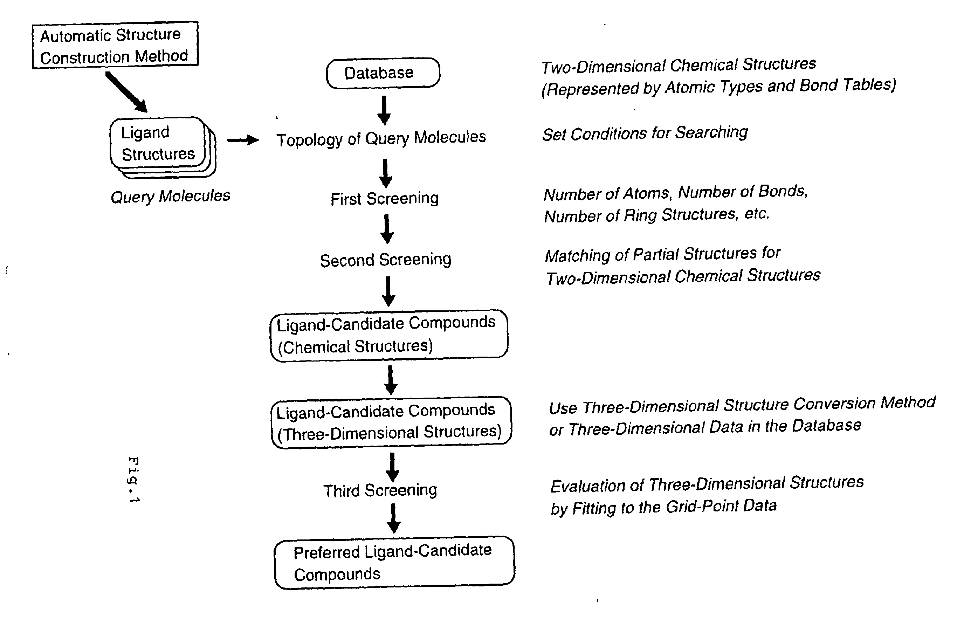

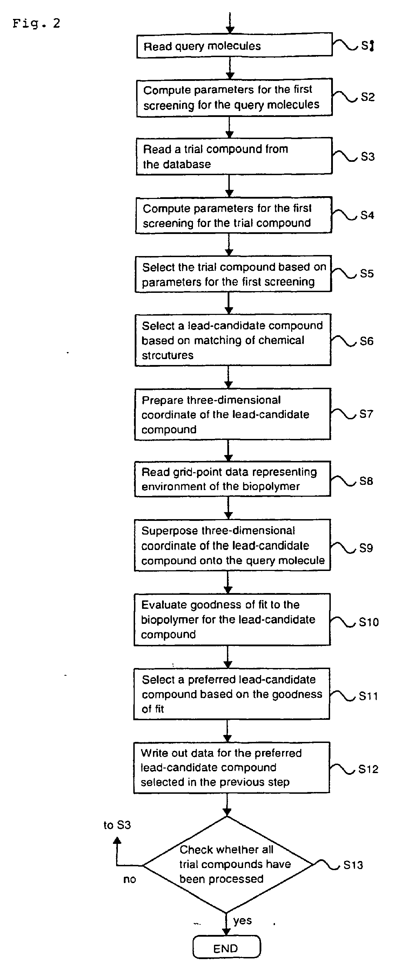

Figure 1 represents an algorithm of a preferred embodiment of the method of the present invention comprising the above steps (a) to (f), and Figure 2 represents the algorithm in more detail (in Figure 2, S represents a step). By referring to these drawings together with the above explanation, it will become easier to understand the present invention, but it should be understood that the scope of the present invention is not limited to these embodiments but is defined by the appended claims.

The lead-candidate compounds obtained as a result of the database searching according to the present invention are those compounds having similarities to the structures of the query molecule in molecular skeleton, molecular shape, interaction with the biopolymer and the like. Those compounds should provide, to a user, information about the molecular structures capable of binding to a target biopolymer, even if modifications such as change of atomic species, addition or deletion of atom or atomic group and the like are applied to the query molecules. If searching is performed for a database of available compounds, selected lead-candidate compounds can be experimentally tested for their activity without synthesizing them. Even if the compounds are not available, one can select compounds preferred from the viewpoints of physiological activity, physical properties (such as solubility), ease of synthetic expansion and the like from much larger number of compounds with much broader variety of structures compared to the query molecules, and then synthesize and confirm their activity.

In order to obtain lead-candidate compounds according to the present invention, information at least about atomic types and mode of covalent bonds is preferable for the query molecules and compounds in a database. If one can use information about marker sites in the query molecules assumed to be essential or important for interaction with the biopolymer, it becomes possible to obtain lead-candidate compounds having broader variety of structures and with higher possibility to act as a ligand.

Furthermore, in order to obtain lead-candidate compounds with higher possibility to bind to the target biopolymer, three-dimensional information of the query molecules is important. Query molecules generated by the automatic structure construction method based on the three-dimensional structure of the target biopolymer or based on the virtual receptor model are considered to contain information such as the active conformation (conformation upon expression of activity through binding to the biopolymer) and the binding scheme to the target biopolymer. When known ligands are used as the query molecules, stable binding schemes and active conformation can also be estimated by fitting them to the target biopolymer and/or the virtual receptor model (for this purpose, the program ADAM: PCT International Publication WO93/20525; M. Y. Mizutani et al., J. Mol. Biol., 243, pp.310-326, 1994 and the program RECEPS: Kato, Y. et al., Tetrahedron Lett., 43, pp.5229-5236, 1987 etc. can be used).

When a database contains information about three-dimensional coordinate (it need not contain information about active conformation, and it is not particularly limited so long as it contains appropriate information such as those about bond distance and bond angle of compounds) in addition to the information of the query molecules mentioned above, one can obtain lead-candidate compounds with higher possibility to act as a ligand, since further selection of the lead-candidate compounds can be performed based on binding schemes to the biopolymer or to the virtual receptor model. The criteria used for such selection may include, for example, binding scheme and its stability, number of hydrogen bonds, number of ionic bonds, and/or hydrophobic bonds.

The method of the present invention can afford more efficient creation of lead compounds, as it enables rapid search for wide range of lead-candidate compounds from enormous number of compounds stored in a compound database, by selecting groups of compounds satisfying requirements for binding to the biopolymer and having equivalent and analogous nature in their interaction, molecular skeleton, molecular shape and the like, based on structure information of molecules that are assumed or confirmed to be capable of binding to the target biopolymer. When query molecules have only information of two-dimensional structures, two-dimensional information about lead-candidate compounds is provided. When query molecules have three-dimensional information such as binding schemes to the biopolymer or to the virtual receptor model, three-dimensional information such as active conformation or binding schemes can be obtained easily for lead-candidate compounds as well. Accordingly, the present invention provides an extremely efficient method for searching a database for compounds that can act as a ligand to a biopolymer, and it can substitute for three-dimensional database searching methods which require huge calculation because of the difficulty of handling of conformational flexibility. The concept of the method of the present invention is shown below.

Structure Generation by
Automatic Structure
Construction Method
and Selection

Structures of Known Ligands
or their Partial Structures

Numerical
Information

Two-Dimensional
Information

Three-Dimensional
Information

Information of
Query Molecules

Various Molecular
Parameters

Atomic Types
Mode of Covalent Bonds
Marker Sites

Atomic Coordinates (Active Conformation)
Marker Sites
Binding Scheme to Biopolymer or Virtual Receptor Model

Information of
Compounds
in Database

Various Molecular
Parameters

Atomic Types
Mode of Covalent Bonds

Atomic Coordinates
(Not Necesarily in Active Conformation)

Selection

Selection

Selection

Information of
Lead-Candidate
Compounds

Molecule Name
Molecule ID

Two-Dimensional
Structures
(Che[?]cal Structures)

Atomic Coordinates (Active Conformation)
Binding Scheme to Biopolymer or Virtual Receptor Model
Stability of the Binding

Lead Candidates

Lead Candidates

### Reference Example

### Example 1

Query molecules were constructed by using LEGEND as the automatic structure construction method, and search of a database containing information of two-dimensional and three-dimensional structures of commercially available compounds, Available Chemicals Directory (MDL Information Systems, Inc., number of stored compounds: 124,000), was performed.

Automated construction of molecular structures was performed for crystal structure of dihydrofolate reductase of lactobacillus (Bolin et al., J. Biol. Chem., 257, p.13650, 1982). The query molecules were constructed under the conditions that the coenzyme NADPH present in the crystal structure was included as a part of the enzyme, and a cavity formed by removing the inhibitor, methotrexate, was considered a ligand binding site. A guanidinium group, which is a partial structure of methotrexate, was used as a partial structure (seed) for the structure construction, and it was placed in the cavity so that it faces the side chain of the Asp-26 in the depth of the cavity. 100 ligands were constructed under the condition that each ligand to be automatically constructed contains 20 atoms at most, and 2 ring structures at least.

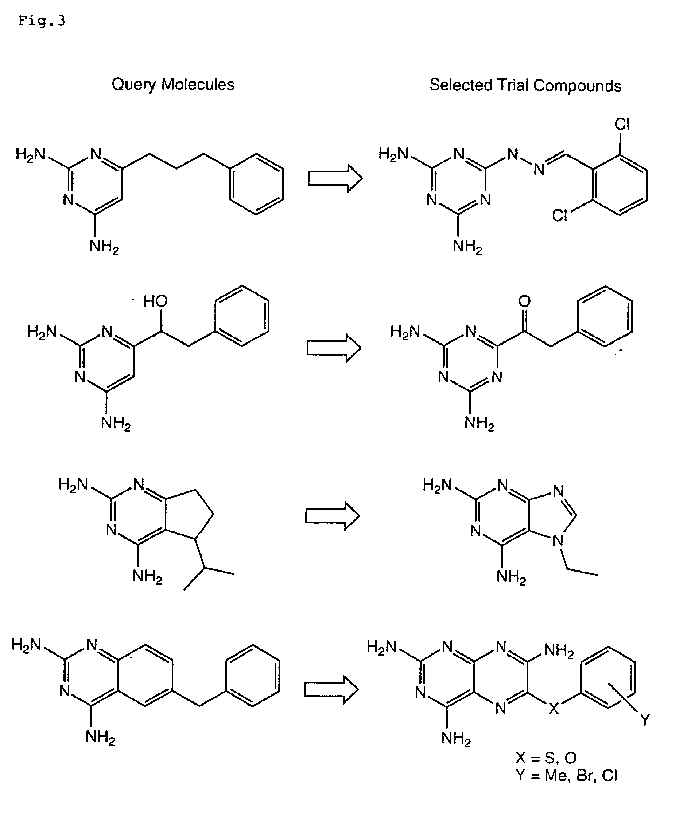

Search of the database was performed by using the constructed ligands as query molecules. The first screening was performed with parameters that were set so that trial compounds having the number of non-hydrogen atoms in a range of lower by one to higher by two compared with the number of non-hydrogen atoms in the query molecules, so that heteroatoms (oxygen atom and nitrogen atom) in the query molecules should be conserved, while carbon atoms in the query molecules may be replaced with other heteroatoms in the trial compounds. The second screening was performed by using the algorithm of Ullmann (Ullmann, J., Assoc. Comput. Mach., 23, p.31, 1976) to finally select 29 lead-candidate compounds. Structures of some of them are shown in Figure 3.

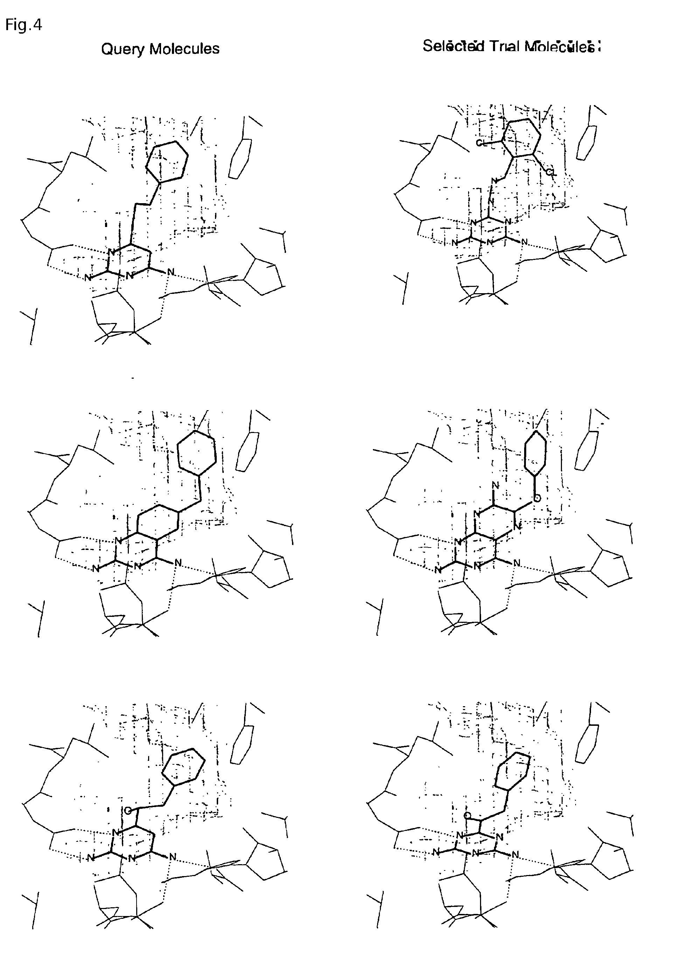

Figure 4 represents comparison of binding schemes to the ligand binding site (cavity) of the biopolymer with respect to the preferred lead-candidate compounds selected by the third screening and the query molecules. The cage-like indications represent a region into which atoms can enter, and molecular structures of the biopolymer are indicated with normal lines, and the structures of query molecules (left) and preferred lead-candidate compounds (right) are indicated with bold lines. From these results, it can be seen that the preferred lead-candidate compounds selected by the method of the present invention completely fit the ligand binding region of the biopolymer, and strongly bind to the biopolymer by effective hydrogen bonds. The compounds selected as the lead-candidate compounds include compounds known to inhibit the activity of dihydrofolate reductase, and hence it was demonstrated that the method of the present invention is useful for the creation of lead compounds for drugs.

### Industrial Applicability

The method of the present invention enables rapid search for lead-candidate compounds capable of binding to a biopolymer by using an ordinary personal computer, workstation or the like, while not requiring huge calculation.

In particular, the method of the present invention concurrently enables estimation of three-dimensional structures of lead-candidate compounds and structures of complexes between a biopolymer and the lead-candidate compounds with active conformation upon binding to the biopolymer. Moreover, lead-candidate compounds selected by the method of the present invention are readily obtainable based on information of a database, and therefore it can advantageously enables easy and rapid determination of propriety of them as lead compounds for drugs without much labor of compound synthesis.

## Claims

1. A computer-based method for selecting lead-candidate compounds capable of binding to a receptor biopolymer from a database containing, at least, information about mode of covalent bonds and three-dimensional structures of compounds, which comprises the following steps:
a) reading at least one query molecule that is known to bind, or expected to be capable of binding, to the biopolymer;
b) selecting one or more lead-candidate compounds from the compound database by matching modes of covalent bonds between the query molecule and trial compounds stored in the database and judging similarity of partial structures of the query molecule and the trial compounds; and
c) estimating a binding scheme of the trial compounds selected as the lead-candidate compounds in step b) to the biopolymer based on three-dimensional information and binding scheme to the biopolymer of the query molecule and based on correspondence of the modes of covalent bonds of the query molecule and the trial compounds selected as the lead-candidate compounds in step b), wherein
said mode of covalent bonds includes information of counterpart atom covalently bonded to a certain atom indicated by input order numbers of the atoms and kind of the chemical bond.

2. The method of claim 1, wherein the database contains information about atomic types; and selecting lead-candidate compounds from the compound database by matching atomic types and modes of covalent bonds between the query molecule and a trial compound stored in the database and judging similarity of partial structures of the query molecule and the trial compound in step b).

3. The method of claims 1 or 2, which further comprises the following steps:
d) calculating one or more parameters relating to interaction between the lead-candidate compound and the biopolymer; and
e) screening the lead-candidate compounds based on the parameters calculated in step d).

4. The method of claims 1 or 2, wherein the biopolymer is a virtual receptor model estimated based on information of three-dimensional structures of one or more known ligands capable of binding to the biopolymer.

5. The method of claim 4, further comprising screening the lead-candidate compounds based on the goodness of fit of the lead-candidate compounds to the virtual receptor model.

6. The method of claims 1, 2, 3, 4 or 5 further comprising constructing the structure of the at least one query molecule by an automatic structure construction method.

## Patentansprüche

1. Ein computerbasiertes Verfahren zum Auswählen von Kandidaten, die als Leitverbindungen dienen, die in der Lage sind, an ein Rezeptor-Biopolymer zu binden, aus einer Datenbank, die wenigstens Informationen über die Art der kovalenten Bindungen und die dreidimensionalen Strukturen der Verbindungen enthält, welches die folgenden Schritte umfasst:
a) Einlesen wenigstens eines fraglichen Moleküls, von dem es bekannt ist, dass es an das Biopolymer bindet, oder von dem erwartet wird, dass es in der Lage ist, an das Biopolymer zu binden;
b) Auswählen eines oder mehrerer Leitverbindungskandidaten aus der Datenbank der Verbindungen durch In-Übereinstimmung-Bringen von Arten kovalenter Bindungen zwischen dem fraglichen Molekül und den zu prüfenden Verbindungen, die in der Datenbank gespeichert sind, und Bewerten der Ähnlichkeit von Teilstrukturen des fraglichen Moleküls und der zu prüfenden Verbindungen; und
c) Abschätzen eines Bindungsschemas der zu prüfenden Verbindungen, ausgewählt als Leitverbindungskandidaten in Schritt b), an das Biopolymer, basierend auf der dreidimensionalen Information und dem Bindungsschema des fraglichen Moleküls an das Biopolymer und basierend auf der Übereinstimmung der Arten von kovalenten Bindungen des fraglichen Moleküls und den zu prüfenden Verbindungen, ausgewählt als die Leitverbindungskandidaten in Schritt b), wobei
die Art der kovalenten Bindungen die Information über ein Gegenatom, daskovalent an ein bestimmtes Atom gebunden ist, angezeigt durch die eingegebenen Ordnungsnummern der Atome und den Typ der chemischen Bindung, einschließt.

2. Das Verfahren gemäß Anspruch 1, wobei die Datenbank Informationen über Atomtypen umfasst und Auswählen der Leitverbindungskandidaten aus der Verbindungsdatenbank durch In-Übereinstimmung-Bringen von Atomtypen und Arten von kovalenten Bindungen zwischen dem fraglichen Molekül und einer zu prüfenden Verbindung, die in der Datenbank gespeichert ist, und Bewerten der Ähnlichkeit von Teilstrukturen des fraglichen Moleküls und der zu prüfenden Verbindung in Schritt b).

3. Das Verfahren gemäß Anspruch 1 oder 2, welches außerdem die folgenden Schritte umfasst:
d) Berechnen eines oder mehrerer Parameter in Bezug auf die Wechselwirkung zwischen dem Leitverbindungskandidaten und dem Biopolymer, und
e) Screening der Leitverbindungskandidaten auf Basis der Parameter, die in Schritt d) berechnet wurden.

4. Das Verfahren gemäß Anspruch 1 oder 2, wobei das Biopolymer ein virtuelles Rezeptormodell ist, das auf Basis von Informationen aus dreidimensionalen Strukturen eines oder mehrerer bekannten Liganden, der/die in der Lage ist/sind an das Biopolymer zu binden, erstellt wurde.

5. Das Verfahren gemäß Anspruch 4, außerdem umfassend das Screening der Leitverbindungskandidaten auf Basis dessen, wie gut die Leitverbindungskandidaten an das virtuelle Rezeptormodell passen.

6. Das Verfahren gemäß Anspruch 1, 2, 3, 4 oder 5, außerdem umfassend das Entwerfen der Struktur wenigstens eines fraglichen Moleküls durch ein automatisches Strukturentwurfsverfahren.

## Revendications

1. Procédé informatisé pour sélectionner des composés candidats-guides capables de se lier à un biopolymère récepteur à partir d'une base de données contenant, au moins, des informations concernant le mode de liaisons covalentes et les structures tridimensionnelles de composés, qui comprend les étapes suivantes :
a) lecture d'au moins une molécule d'interrogation dont on sait qu'elle se lie, ou dont on s'attend à ce qu'elle soit capable de se lier, au biopolymère ;
b) sélection d'un ou plusieurs composés candidats-guides à partir de la base de données de composés en appariant des modes de liaisons covalentes entre la molécule d'interrogation et des composés d'essai stockés dans la base de données et en évaluant la similarité de structures partielles de la molécule d'interrogation et des composés d'essai ; et
c) estimation d'un schéma de liaison des composés d'essai sélectionnés comme composés candidats-guides dans l'étape b) au biopolymère sur la base des informations tridimensionnelles et du schéma de liaison au biopolymère de la molécule d'interrogation et sur la base de la correspondance des modes de liaisons covalentes de la molécule d'interrogation et des composés d'essai sélectionnés comme composés candidats-guides dans l'étape b), où
ledit mode de liaisons covalentes inclut des informations d'un atome homologue lié de manière covalente à un certain atome indiqué par des numéros d'ordre d'entrée des atomes et le type de la liaison chimique.

2. Procédé selon la revendication 1 où la base de données contient des informations concernant les types atomiques ; et la sélection de composés candidats-guides à partir de la base de données de composés en appariant des types atomiques et des modes de liaisons covalentes entre la molécule d'interrogation et un composé d'essai stocké dans la base de données et l'évaluation de la similarité de structures partielles de la molécule d'interrogation et du composé d'essai dans l'étape b).

3. Procédé selon la revendication 1 ou 2 qui comprend en outre les étapes suivantes :
d) calcul d'un ou plusieurs paramètres liés à une interaction entre le composé candidat-guide et le biopolymère ; et
e) criblage des composés candidats-guides sur la base des paramètres calculés dans l'étape d).

4. Procédé selon la revendication 1 ou 2 où le biopolymère est un modèle de récepteur virtuel estimé sur la base des informations des structures tridimensionnelles d'un ou plusieurs ligands connus capables de se lier au biopolymère.

5. Procédé selon la revendication 4 comprenant en outre le criblage des composés candidats-guides sur la base de la qualité de l'ajustement des composés candidats-guides au modèle de récepteur virtuel.

6. Procédé selon les revendications 1, 2, 3, 4 ou 5 comprenant en outre la construction de la structure de la au moins une molécule d'interrogation par un procédé de construction de structure automatique.
